# EUROPÄISCHE PATENTSCHRIFT

(11) **EP 2 349 427 B1**
(45) Veröffentlichungstag und Bekanntmachung des Hinweises auf die Patenterteilung: **12.06.2013**
(21) Anmeldenummer: 09759868.4
(22) Anmeldetag: 20.11.2009
(51) Int. Cl.: A61M 16/04

(54) **LARYNXMASKE MIT OESOPHAGEALDURCHGANG**
LARYNX MASK HAVING AN ESOPHAGEAL PASSAGE
MASQUE LARYNGÉ POURVU D'UN PASSAGE OESOPHAGIEN

(30) Priorität: 27.11.2008 CH 18592008
(43) Veröffentlichungstag der Anmeldung: 03.08.2011
(73) Patentinhaber: Deltona Innovations AG, 8124 Maur (CH)
(72) Erfinder: DUBACH, Werner, F., 8124 Maur (CH)
(74) Vertreter: Schneider Feldmann AG Patent- und Markenanwälte
(86) Internationale Anmeldenummer: PCT/CH2009/000373
(87) Internationale Veröffentlichungsnummer: WO 2010/060226

(56) Entgegenhaltungen:
- EP-A- 1 875 937
- WO-A-2006/125986
- WO-A1-2005/011784
- GB-A- 2 404 863
- US-A- 5 878 745
- US-A1- 2003 037 790
- US-B2- 7 040 322

## Beschreibung

Die vorliegende Erfindung betrifft eine zur Einführung durch den mittleren Rachenraum über den Kehlkopfdeckel geeignete Larynxmaske, umfassend eine Deckplatte mit einem Tubuseinführungsstutzen, einer Respirationslumenmündung und einem Oesophagealdurchgang sowie einem die Deckplatte an der ventralen Seite umlaufenden, aufblasbaren Cuff. Larynxmasken werden mittels supraglottischen Tuben, welche zur Offenhaltung der Atemwege und zur Beatmung eines Patienten in den Rachenraum eingeführt werden, angeboten. Mittels des supraglottischen Tubus wird eine Kehlkopfmaske durch den mittleren Rachen über den Kehlkopf in den unteren Rachenraum eingeführt und hinter beziehungsweise um den Kehlkopf platziert. Solche Larynxmasken dienen der Beatmung eines Patienten, während dieser anästhesiert ist. Sie erlauben auch das Einführen von Tuben, Sonden, optischen Instrumenten und anderen Instrumenten in die Atemwege. Immer häufiger verfügen solche Larynxmasken über einen oesophagealen Zugang. Dieser erlaubt das Einführen von Sonden in die Speiseröhre und in den Magen, um Magensaft und andere Flüssigkeiten sowie Luft aus dem Magen abzusaugen. Das Entleeren des Magens bei anästhesierten Patienten soll verhindern, dass Mageninhalt in die oberen Atemwege zurückfliesst und in die ungeschützten Atemwege (Luftröhre, Bronchien und Lungen) aspiriert wird. Ein weiterer Vorteil eines oesophagealen Zugangs ist das Entweichen von passiv oder aktiv regurgitiertem Mageninhalt aus dem oberen Oesophagus nach aussen, und stellt somit einen begrenzten, und damit ungenügenden Aspirationsschutz dar. Diese Larynxmasken erlauben aber nicht das Absaugen von Flüssigkeiten aus dem Rachenraum.

Auf dem Markt ist eine grosse Anzahl unterschiedlicher Larynxmasken bekannt. Ein typisches Beispiel zeigt die US 5878745. Hier ist eine Gastro-Laryngealmaske gezeigt, bei der der supraglottische Tubus ein Rohr ist, durch das mehrere Schläuche geführt werden. Diese Schläuche haben Lumen, die für die Beatmung und für einen oesophagealen Zugang dienen.

Das Einführen einer Larynxmaske ist nicht immer einfach. Larynxmasken mit einem relativ starren supraglottischen Tubus lassen sich einfacher einführen, wobei die Steifigkeit eine Anpassung der Position der Larynxmaske an die anatomischen Gegebenheiten verhindert. Die Einführung mittels relativ starren supraglottischen Tuben in den Rachenraum können zu Verletzungen führen und die Positionierung im Rachenraum ist nicht immer zuverlässig.

Hochflexible supraglottische Tuben mit relativ weichen Larynxmasken Cuff erlauben eine bessere Positionierung der Larynxmaske um den Kehlkopf, sind aber schwieriger und damit gelegentlich traumatisch zum Einführen und schwieriger in der Positionierung im Rachenraum. Entsprechend kommt es immer wieder vor, dass bei solchen hochflexiblen supraglottischen Tuben mit weichen Larynxmaskencuff bei der Einführung das proximale Ende der Larynxmaske, insbesondere die sogenannte Spitze umgeknickt wird. Damit ist eine zuverlässige Dichtung der Larynxmaske um den Kehlkopf nicht mehr gegeben. Um dieses Problem zu beheben, kann man auf ein biegesteiferes Material ausweichen, wobei dann allerdings die Vorteile der hochflexiblen Materialien verloren gehen. Traumatische Effekte im mittleren Rachenraum sind entsprechend die Folge. Auch mit einem leicht erhöhten Luftdruck im Cuff lässt sich das Problem nicht sicher lösen. Auch wird bei den heute bekannten Larynxmasken der Oesophagealdurchgang immer durch den Cuff hindurch geführt. Dies kompliziert die gesamte Herstellung der Larynxmaske. Tritt eine Knickung oder auch nur eine etwas stärkere Umbiegung der Spitze der Larynxmaske auf, so ist damit der Oesophagealdurchgang meist schon nicht mehr frei und ein Instrumentarium oder eine Sonde lässt sich kaum mehr einführen.

Es sind eine Vielzahl von Larynxmasken mit einem oesophagealen Durchgang bekannt. Der oesophageale Durchgang endet in einem oesophagealen Ausgang der am äussersten proximalen Ende der Larynxmaske angeordnet ist. Hierbei muss nun der oesophageale Durchgang durch den umlaufenden Dichtbereich der Larynxmaske geführt werden. Handelt es sich bei einer Larynxmaske um eine Version ohne aufblasbaren Cuff, wie in den Dokumenten EP 1 875 937 oder GB 2 404 863 gezeigt, so ist dies relativ unproblematisch da hier die Larynxmaske insgesamt wesentlich steifer gestaltet sind und ein Knicken der Spitze praktisch kein relevantes Problem darstellt.

Wesentlich komplexer ist die Situation bei Larynxmasken mit aufblasbarem Cuff. Einerseits ist die Spitze der Larynxmaske wegen der Dünnwandigkeit des Cuffs sehr flexibel und damit knickanfällig und andererseits ist die Durchführung des oesophagealen Durchgangs durch den Cuff hindurch sehr problematisch. Diese Problematik der Durchführung durch den Cuff löst die WO 2006/125 986 mit einer komplexen vielteiligen Larynxmaske, einem separaten Tubus als oesophagealen Durchgang, der durch die Deckplatte und den Trachealtubus nachträglich einschiebbar ist.

Ähnlich ist die Konstruktion offenbart in der US 2004/0020491. Zusätzlich wird hier der durchgeschobene separate Schlauch des oesophagealen Durchgangs mit einem separaten Cuff gedichtet.

Die Lösung mit separatem Schlauch als oesophagealen Durchgang versteifen zwar die Larynxmaske insgesamt, so dass eine Knickgefahr der Spitze geringer ist, doch wird die gesamte Konstruktion aufwendig und unhandlicher. Dies trifft auch auf die Lösung gemäss der US 5 878 745 zu.

Letztlich ist noch aus der US 2003/0037790 eine Larynxmaske bekannt mit aufblasbaren Cuff, wobei hier der geschlossene oesophagealeDurchgang über den Cuff hinweg geschlossen geführt wird und der Ausgang am proximalen Ende der Spitze jenseits des aufblasbaren Cuffs liegt. Dieser mehrfach gekrümmte geschlossene Verlauf verunmöglicht praktisch die Verwendung des oesophagealen Durchgangs zur Instrumentierung.

Es ist daher die Aufgabe der vorliegenden Erfindung eine Larynxmaske derart zu verbessern, dass auch bei Verwendung eines hochflexiblen Materials die genannten Probleme nicht mehr auftreten, respektive deren Auftreten wesentlich reduziert wird, sowie das Entweichen von regurgitierten Speisen und Flüssigkeiten in den dorsalen Rachenraum zu ermöglichen. Des weiteren soll eine verbesserte Verwendung des oesophagealen Durchganges zur Instrumentierung ermöglicht werden.

Diese Aufgabe löst eine Larynxmaske gemäss Oberbegriff des Patentanspruches 1 die sich dadurch auszeichnet, dass der Oesophagealdurchgang am proximalen Ende der Deckplatte auf der dorsalen Seite austritt und über den Cuff als offener Kanal geführt ist, wobei beidseitig dieses Kanals und in axialer Richtung des Kanals wirksame Versteifungsmittel vorhanden sind, die einer möglichen Knickung der proximalen Spitze der Larynxmaske entgegenwirken.

Weitere vorteilhafte Ausgestaltungsformen gehen aus den abhängigen Patentansprüchen hervor und deren Bedeutung und Wirkungsweise sind in der nachfolgenden Beschreibung mit Bezug auf die anliegenden Zeichnungen erläutert.

In den Zeichnungen sind zwei bevorzugte Ausführungsvarianten des Erfindungsgegenstandes dargestellt, wobei die Figuren 1 bis 7 eine erste Ausführungsform und die Figuren 8 bis 10 eine zweite Ausführungsform zeigen.

Es zeigt:
- Fig. 1: eine perspektivische Gesamtansicht der erfindungsgemässen Larynxmaske mit einem daran angeschlossenen supraglottischen Tubus mit Anschlussstutzen;
- Fig. 2: die Larynxmaske in einer ventral-distalen Ansicht und
- Fig. 3: wiederum eine perspektivische Darstellung, jedoch von der dorsal-proximalen Seite gesehen.
- Fig. 4: stellt einen diametralen Längsschnitt durch die Larynxmaske dar, in der
- Fig. 5: ist ein Vertikalschnitt durch die Larynxmaske im Bereich nahe des Tubuseinführungsstutzens mit Blickrichtung zum proximalen Ende und
- Fig. 6: eine etwa mittige Querschnittsdarstellung mit Blickrichtung zum distalen Ende; während
- Fig. 7: wiederum einen Vertikalschnitt nahe dem proximalen Ende der Deckplatte mit Blickrichtung zum proximalen Ende darstellt.
- Fig. 8: zeigt die Larynxmaske in einer zweiten Ausführungsform in einer Aufsicht auf die dorsale Seite, während
- Fig. 9: einen senkrechten Schnitt durch die Larynxmaske im Bereich deren Spitze zeigt. Schliesslich ist in
- Fig. 10: nochmals eine perspektivische Ansicht der zweiten Ausführungsform der Larynxmaske dargestellt.

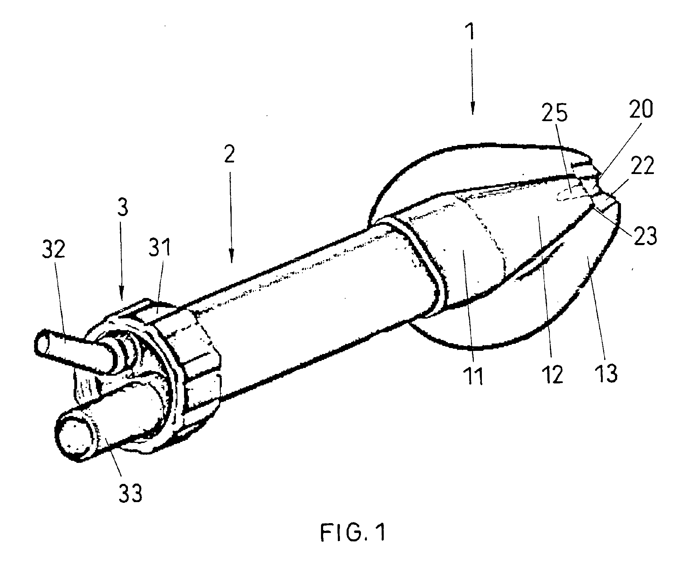

In der Figur 1 erkennt man die Larynxmaske 1 mit einem daran angeschlossenen supraglottischen Tubus 2, während am anderen Ende ein Anschlussstutzen 3 befestigt ist. Zur Verbindung der Larynxmaske 1 mit dem supraglottischen Tubus 2 weist die Larynxmaske am distalen Ende einen Tubuseinführungsstutzen 11 auf. Dieser Tubuseinführungsstutzen 11 ist einstückig mit der direkt daran anschliessenden Deckplatte 12 verbunden, an der wiederum einstückig angeformt ein aufblasbarer Kragen, der sogenannte Cuff 13, angeformt ist, welcher die Deckplatte 12 vollständig umlaufend an dessen ventralen Peripherie umgibt. Der supraglottische Tubus 2 kann zwei Schläuche enthalten oder wie hier bevorzugterweise mit mindestens zwei getrennten Lumen versehen sein. Hierbei ist das eine Lumen als Respirationslumen verwendet, während das zweite Lumen als Instrumental- oder Oesophageallumen ausgebildet ist. Dazwischen kann ein drittes Lumen vorhanden sein, mittels dem der supraglottische Tubus 2 mittels entsprechend darin gelagerten Zug- und Druckmittel in seiner Krümmung veränderbar ist. In der Figur 1 ist jedoch der supraglottischen Tubus in einer geraden Ausgangsposition gezeigt. Die Verstellbarkeit bzw. Veränderung der Krümmung des supraglottischen Tubus 2 lässt sich durch Verdrehen eines Stellringes 31 realisieren. Aus dem Anschlussstutzen 3 ragt einerseits ein Oesophagealeingangsstutzen 32 und andererseits ein Respirationseingangsstutzen 33. Bezüglich der weiteren Merkmale der Larynxmaske 1 wird auf die nachfolgenden Figuren verwiesen.

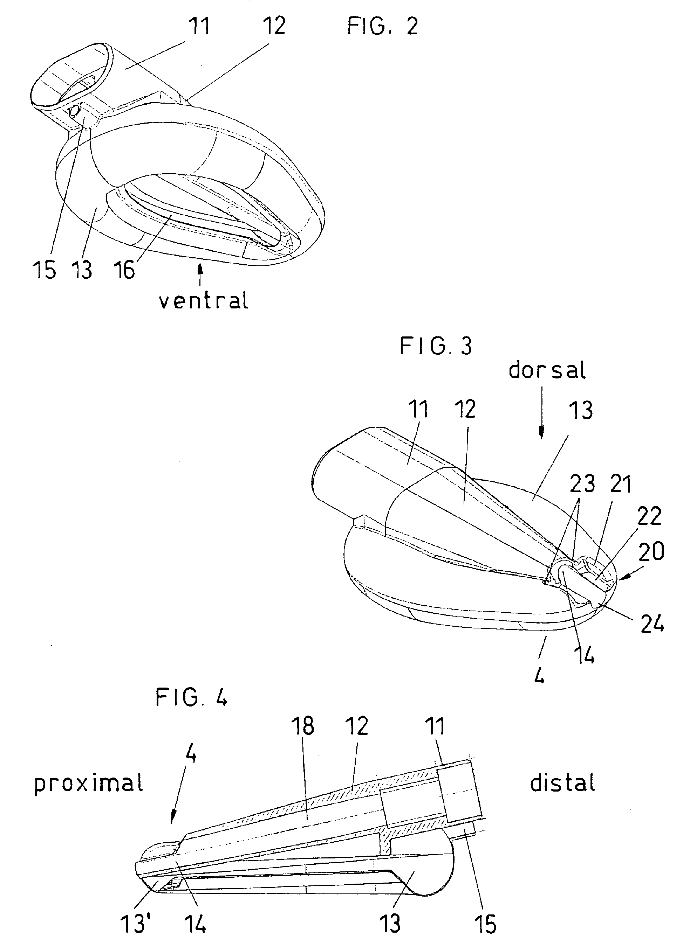

In der Figur 2 ist die Larynxmaske 1 perspektivisch in einer Ansicht von ventral-distal dargestellt. Hier erkennt man den Tubuseinführungsstutzen 11 und blickt teilweise in diesen hinein, so dass hier ein Teilblick in den Oesophagealdurchgang 18 ersichtlich ist. In sämtlichen Figuren ist die Larynxmaske 1 in der Form dargestellt, wie sie aus einer entsprechenden Kunststoffspritzform kommt. Der innere Rand des Cuff's 13 ist hier noch nicht mit einer inneren umlaufenden Klebewand 16 verbunden. Auch wenn hier von einer Klebewand 16 gesprochen wird, so kann die Verbindung des Randes des Cuffs 13 mit der Klebewand 16 nicht nur durch Kleben sondern auch durch Schweissen erfolgen. Insbesondere eine Ultraschallverschweissung dürfte hier in Frage kommen. Durch die Elastizität des Materials scheint der Cuff 13 in seiner Gestalt praktisch gleich, wie wenn dieser bereits mit Luft gefüllt wäre. Für die Zuführung der Füllluft in den Cuff 13 ist unterhalb des Tubuseinführungsstutzens 11 ein Belüftungsstutzen 15 angeformt, der direkt in den Cuff 13 mündet, wie dies am deutlichsten im Vertikalschnitt der Figur 4 ersichtlich ist.

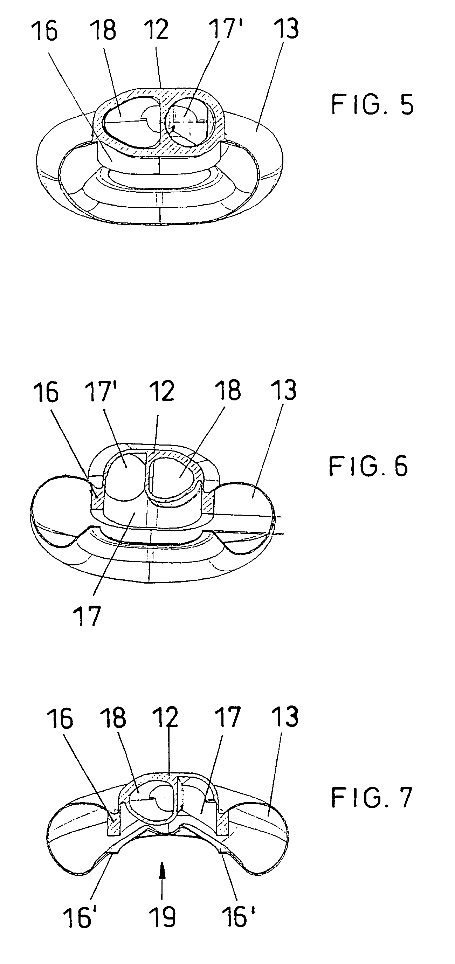

Innerhalb der Deckplatte 12 sind im Bereich nahe des Tubuseinführungsstutzens 11 das Respirationslumen 17 anfänglich noch vollständig im Querschnitt geschlossen, genauso wie das Lumen des Oesophagealdurchganges 18. Entsprechend wird hier der Respirationslumen mit 17' bezeichnet. Der Vertikalschnitt nach Figur 5 ist in Blickrichtung zur Spitze 4 bzw. zum proximalen Ende der Larynxmaske gezeigt. Entsprechend sieht man beim Blick in den Oesophagealdurchgang 18 den Oesophagealausgang 14.

In Verlaufrichtung von distal zu proximal öffnet sich das Respirationslumen 17' immer weiter und bildet so die Respirationslumenmündung 17 und kommuniziert mit dem darunter liegenden und vom Cuff 13 begrenzten, gedichteten Respirationsraum 19. Während in der Figur 6 der Vertikalschnitt mit Blickrichtung zum distalen Ende gezeigt ist, so dass hier der freie Durchgang des Respirationslumens 17' bis zu dessen Austritt im Tubuseinführungsstutzen 11 ersichtlich ist, während gleichzeitig auch die Respirationslumenmündung 17 ersichtlich ist. In der Figur 7 ist nochmals ein Vertikalschnitt gezeigt, relativ nahe des proximalen Endes bzw. nahe der Spitze 4 der Larynxmaske 1, wiederum in Blickrichtung zum proximalen Ende. Deutlich erkennbar sind wiederum die Klebewand 16 und der entsprechende Kleberand 16' am Cuff 13 der hier noch nicht mit der Klebewand 16 verbunden ist.

Der lateral ventral neben der Respirationslumenmündung 17 verlaufende Oesophagealdurchgang 18 hilft einerseits die Larynxmaske in axialer Richtung zu verstreben, um Knickung im Mittelbereich der Maske zu verhindern bzw. Zu reduzieren und anderseits hilft der Oesophagealzugang 18 auf der Ventralseite, den Kehldeckel von Respirationslumenmündung 17 weg zu halten.

Vorzugsweise verfügt die Larynxmaske zwischen Oesophagealdurchgang 18 und Respirationslumen 17' und Respirationslumenöffnungen 17 ein zusätzliches gegen proximal abgeschlossenes sackartiges Lumen, welches das Einsetzen eines Längsversteifungselements erlaubt.

Das proximale Ende der Larynxmaske 1 bildet deren Spitze 4. Im Bereich der Spitze 4 der Teil der Deckplatte 12 bildet mündet der geschlossen durch die Deckplatte 12 verlaufende Oesophagealdurchgang 18 in den Oesophagealausgang 14. Der Oesophagealdurchgang 18 verläuft dann über den Cuff 13 hinweg als gegen dorsal offener Kanal 20. Der Querschnitt dieses Kanals 20 ist hier als Halbzylinder gebildet. Entsprechend ist der Cuff 13 im Bereich der Spitze 4 im Querschnitt reduziert, da die Deckplatte 12 hier in Richtung ventral tiefer verläuft. Der reduzierte Querschnittsbereich des Cuffs 13 ist mit 13' bezeichnet. Prinzipiell kann der Querschnitt des Cuffs in diesem Bereich auf Null reduziert sein, so dass der Cuff im Bereich der Spitze unterbrochen ist. Die gezeigte Lösung wird jedoch wegen der besseren Abdichtung zum Kehlkopfdeckel bevorzugt. Im Prinzip könnte direkt anschliessend am halbzylindrischen offenen Kanal 20 die Begrenzungswände zum Cuff 13 verlaufen. Diese Begrenzungswände sind prinzipiell als Versteifungsmittel 21 der Deckplatte 12 gestaltet. Unabhängig davon, ob diese Wände versteift bzw. im Querschnitt verdickt gestaltet sind oder nicht, wirken sie allein durch ihre Verlaufsrichtung senkrecht zur möglichen Knickrichtung als Versteifung. Dies trifft auch dann zu, wenn diese Wände direkt angrenzend am offenen Kanal 20 angeordnet sind.

Bevorzugterweise werden jedoch diese Versteifungswände 21' als Versteifungsmittel 21 gegenüber dem offenen Kanal 20 mit seinem Kanalboden 24 lateral versetzt angebracht. Durch diese laterale Versetzung bildet sich eine Kanalverbreiterung 22 in Form von Zwischenböden. Zwar wäre es auch möglich, diese Zwischenböden durch eine entsprechende Materialverdickung zu verstärken, doch hat dies wenig Effekt im Sinne einer Verhinderung der Knickbildung der Spitze 4.

Durch die Versetzung der Versteifungswände 21' bilden sich seitlich im Übergangsbereich der Deckplatte 12 zum Cuff 13 am distalen Ende dieses erweiterten Bereiches praktisch beidseitig je eine Entweichungsstelle 23. Sollte bei einem Regurgitieren des Mageninhalts dieser nicht durch den Oesophagealdurchgang allein innert genügender Zeit aufgenommen werden können, so kann auch bei den dorsalen Entweichungsstellen 23 dieser Mageninhalt nach dorsal des Cuffs in den Rachenraum entweichen, ohne dass dabei die Gefahr besteht, dass das Material ventral des Cuffs hindurch in den Respirationsbereich gelangt. Weiter kann sich im Bereich des offenen Kanals 20 Rachensekret ansammeln, welches durch das Oesophagealdurchgang hindurch direkt oder mittels einer Sonde abgesaugt werden kann.

In einer weiteren bevorzugten Ausführung können die Versteifungswände 21' weiter als nur im Cuffspitzenbereich entlang der dorsalen Cuffinnenseite nach distal reichen, sodass das Entweichen von Mageninhalt nach dorsal noch effizienter wird und nicht nur die Cuffspitze sondern auch der distale Cuff gegen Knicken geschützt wird.

In einer weiteren bevorzugten Ausführung kann der Oesophagealausgang 14 noch weiter gegen distal sein, sodass der offene Kanal 22, 24 nicht nur über die Cuffspitze beschränkt verläuft. Dies erlaubt ein noch effizienteres Entweichen von regurgitiertem Mageninhalt nach dorsal in den Rachenraum und erleichtertes Absaugen von angesammeltem Rachensekret. Diese erweiterte Absaugöffnung ist mit 25 bezeichnet und strichliniert in der Figur 1 eingefügt.

Der erweiterte Kanal besitzt zudem den Vorteil, dass die Beweglichkeit eines Instrumentes einer Sonde oder einer entsprechenden Optik die durch das Oesophagealdurchgang hindurch geführt wird, bereits frühzeitig eine erforderliche Krümmung durchführen kann, um so, wie gewünscht, in den Oesophagus einzuführen. Wird ein Oesophagealdurchgang durch den Cuff hindurchgeführt, wie dies in den meisten bekannten Lösungen des Standes der Technik geschieht, so wird der Austrittspunkt in proximaler Richtung weiter nach ventral verlegt und entsprechend kann bei gewissen Umständen die erforderliche Hantierung erschwert werden.

Mit der reduzierten Cuffspitze mit offenen Kanal wird bewusst auf eine Keilbildung der Larynxmaske im oberen Oesophaguseingang verzichtet. Die hat nebst den oben genannten Vorteilen weiter den Vorzug, dass der nasooesophageale oder naso-gastrische Zugang eine entsprechende Instrumentierung oder Einlage von transienten oder permanenten Sonden erlaubt.

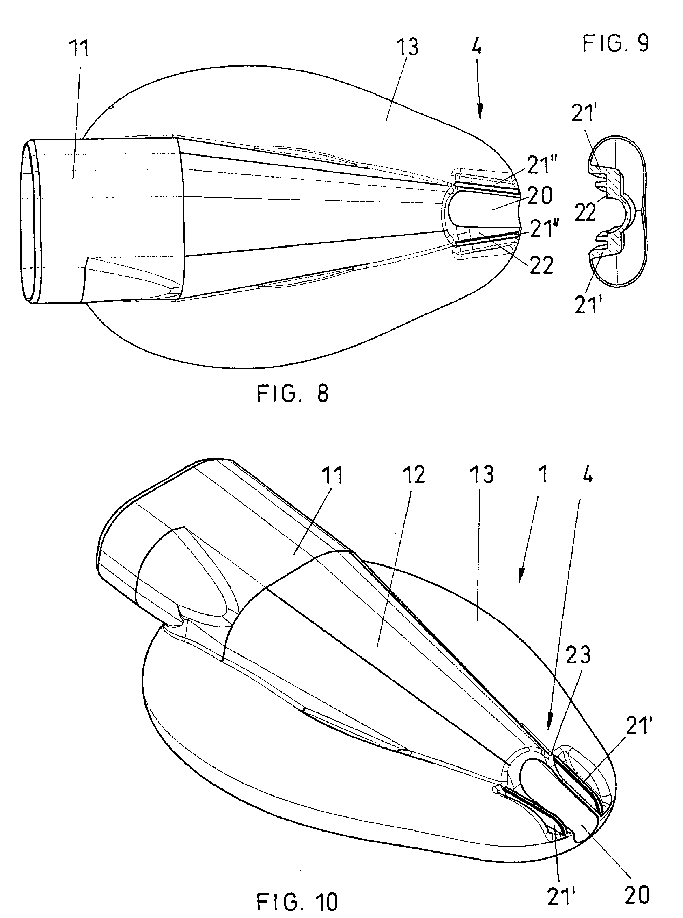

Als zusätzliche Möglichkeit der Versteifung der Spitze 4 der Larynxmaske 1 ist eine weitere Lösung in den Figuren 8 bis 10 dargestellt. Da der Oesophagealdurchgang nicht exakt zentrisch in Längsrichtung verläuft, verläuft entsprechend dessen Fortsetzung, nämlich der offene Kanal 20, ebenfalls nicht zentrisch sondern leicht von lateral zum Zentrum hin. Auch bei der hier dargestellten Lösung erkennt man die Kanalverbreiterung 22. Zusätzlich sind hier auf die Kanalverbreiterung 22, die hier auch als verdickten Wandabschnitt gestaltet ist, zwischen den ersten Versteifungsmitteln den Versteifungswänden 21' zusätzlich auf der Kanalverbreiterung 22 parallel zu den Wänden 21' verlaufend Versteifungsrippen 21" angebracht. Hierdurch wird eine optimale Versteifung gegen den Knickeffekt erreicht. Ein mögliches Entweichen vom Mageninhalt ist dadurch trotzdem noch sichergestellt.

Die erfindungsgemässe Larynxmaske bietet somit, einerseits für den Anästhesisten eine höhere Sicherheit gegen ein mögliches Knicken der Spitze 4 der Larynxmaske, wodurch dieser schneller und sicherer arbeiten kann, und bietet gleichzeitig einen verbesserten Zugang zum Oesophagus, dank des früheren weiter in distaler Richtung gelegenen Oesophagealaustrittes 14 und der dadurch erreichten, höheren Beweglichkeit der entsprechenden, durch den Oesophagealdurchgang hindurchgeführten Instrumenten, Sonden oder optischen Systemen.

### Bezugszeichenliste:

| | |
|---|---|
| 1 | Larynxmaske |
| 2 | supraglottischen Tubus |
| 3 | Anschlussstutzen |
| 4 | Spitze einer Larynxmaske |
| 11 | Tubuseinführungsstutzen |
| 12 | Deckplatte |
| 13 | Cuff |
| 13' | Cuff im Bereich der Spitze |
| 14 | Oesophagealausgang |
| 15 | Belüftungsstutzen des Cuff |
| 16 | Klebewand |
| 16' | Kleberand |
| 17 | Respirationslumenmündung |
| 17' | Respirationslumen |
| 18 | Oesophagealdurchgang |
| 19 | gedichteter Respirationsraum |
| 20 | offener Kanal |
| 21 | Versteifungsmittel |
| 21' | Versteifungswände |
| 21" | Versteifungsrippen |
| 22 | Kanalverbreiterung |
| 23 | Entweichstellen |
| 24 | Boden des Kanals |
| 25 | Erweiterte Absaugöffnung |
| 31 | Verstellring |
| 32 | Oesophagealeingangsstutzen |
| 33 | Respirationseingangsstutzen |

## Patentansprüche

1. Zur Einführung einer durch den mittleren Rachen über den Kehlkopfdeckel geeignete Larynxmaske (1), umfassend eine Deckplatte (12) mit einem Tubuseinführungsstutzen (11), einer Respirationslumenmündung (17) und einem Oesophagealdurchgang (18) sowie einem die Deckplatte an der ventralen Seite umlaufenden, aufblasbaren Cuff, wobei die Deckplatte proximal sich über den Cuff hinweg erstreckt, **dadurch gekennzeichnet, dass** der Oesophagealdurchgang (18) vor dem proximalen Ende der Deckplatte auf der dorsalen Seite aus der Deckplatte austritt und über den Cuff (13) in der Deckplatte als offener Kanal (20) geführt ist, wobei beidseitig dieses Kanals (20) und mindestens annähernd in axialer Richtung desselben wirksame Versteifungsmittel (21, 21', 21") vorhanden sind, die einer möglichen Knickung der proximalen Spitze der Larynxmaske (4) entgegenwirken.

2. Larynxmaske nach Anspruch 1, **dadurch gekennzeichnet, dass** der Cuff (13) im Bereich der proximalen Spitze (4) der Larynxmaske (1) bezüglich der lichten Weite um die Tiefe des Kanals (20) reduziert ist.

3. Larynxmaske nach Anspruch 1, **dadurch gekennzeichnet, dass** der Cuff (13) im Bereich der proximalen Spitze (4) der Larynxmaske unterbrochen ist.

4. Larynxmaske nach Anspruch 2, **dadurch gekennzeichnet, dass** der Boden (24) des Kanals (20) durch den ventralen Bereich des Oesophagealdurchganges (18) gebildet ist.

5. Larynxmaske nach Anspruch 1, **dadurch gekennzeichnet, dass** der Cuff (13) seitlich des Kanals (20) durch ventro-dorsal verlaufende Versteifungswände (21') begrenzt ist und hierdurch die Spitze (4) der Larynxmaske (1) gegen Knickung versteift ist.

6. Larynxmaske nach Anspruch 5, **dadurch gekennzeichnet, dass** die Versteifungswände (21') dicker sind als die angrenzenden Bereiche des Cuffs (13).

7. Larynxmaske nach Anspruch 5, **dadurch gekennzeichnet, dass** die Versteifungswände (21') parallel zum Kanal (20) um eine Distanz versetzt verlaufend angeordnet sind.

8. Larynxmaske nach Anspruch 7, **dadurch gekennzeichnet, dass** zwischen dem Kanal (20) und den Versteifungswänden (21') beidseitig des Kanals ein gegenüber dem Boden (24) des Kanals (20) je ein Absatz als Kanalverbreiterung vorhanden ist.

9. Larynxmaske nach Anspruch 8, **dadurch gekennzeichnet, dass** auf den versetzten Absätzen jeweils mindestens eine weitere Versteifungsrippe (21") angeformt ist.

10. Larynxmaske nach Anspruch 1, **dadurch gekennzeichnet, dass** die Tiefe des Kanals (20) mindestens annähernd einen bis zwei Drittel des Durchmessers des Oesophagealdurchganges (18) beträgt.

11. Larynxmaske nach Anspruch 7, **dadurch gekennzeichnet, dass** beim Übergang des Cuffs (13) zur Deckplatte (12) am distalen Ende der Kanalverbreiterung (22) Entweichungsstellen (23) gebildet sind.

12. Larynxmaske nach Anspruch 1, **dadurch gekennzeichnet, dass** die Versteifungswänden (21') beidseitig vom Cuff-Spitzenbereich weiter entlang der dorsalen Cuffinnenseite geführt sind.

13. Larynxmaske nach Anspruch 1, **dadurch gekennzeichnet, dass** der Oesophagealdurchgang auch teilweise im Deckplattenbereich offen ist und so eine erweiterte Absaugöffnung (25) bildet.

## Claims

1. A larynx mask (1) suitable for insertion through the middle of the pharynx via the epiglottis, said mask comprising a cover plate (12) with a tube insertion connector (11), a respiration lumen opening (17), and an oesophageal passage (18) and an inflatable cuff peripherally surrounding the cover plate on the ventral side, the cover plate extending proximally beyond the cuff, **characterised in that** the oesophageal passage (18) emerges from the cover plate before the proximal end of the cover plate on the dorsal side and is passed over the cuff (13) as an open channel (20) in the cover plate, reinforcing means (21, 21', 21") being provided on both sides of this channel (20), acting approximately in the axial direction thereof and counteracting possible kinking of the proximal tip of the larynx mask (4).

2. The larynx mask according to Claim 1, **characterised in that**, in the region of the proximal tip (4) of the larynx mask (1), the cuff (13) is reduced with regard to the clear width by the depth of the channel (20).

3. The larynx mask according to Claim 1, **characterised in that** the cuff (13) is interrupted in the region of the proximal tip (4) of the larynx mask.

4. The larynx mask according to Claim 2, **characterised in that** the base (24) of the channel (20) is formed by the ventral region of the oesophageal passage (18).

5. The larynx mask according to Claim 1, **characterised in that** the cuff (13) laterally of the channel (20) is delimited by reinforcing walls (21') running ventro-dorsally and the tip (4) of the larynx mask (1) is thus reinforced against kinking.

6. The larynx mask according to Claim 5, **characterised in that** the reinforcing walls (21') are thicker than the adjoining regions of the cuff (13).

7. The larynx mask according to Claim 5, **characterised in that** the reinforcing walls (21') are arranged parallel to the channel (20), offset by a distance.

8. The larynx mask according to Claim 7, **characterised in that**, between the channel (20) and the reinforcing walls (21'), a shoulder is provided on each side of the channel as a channel widening, opposite the base (24) of the channel (20).

9. The larynx mask according to Claim 8, **characterised in that** at least one further reinforcing rib (21'') is integrally formed on the offset shoulders.

10. The larynx mask according to Claim 1, **characterised in that** the depth of the channel (20) is at least approximately one third to two thirds of the diameter of the oesophageal passage (18).

11. The larynx mask according to Claim 7, **characterised in that** escape points (23) are formed at the transition of the cuff (13) to the cover plate (12) on the distal end of the channel widening (22).

12. The larynx mask according to Claim 1, **characterised in that** the reinforcing walls (21') are continued along the dorsal inner side of the cuff on both sides of the cuff tip region.

13. The larynx mask according to Claim 1, **characterised in that** the oesophageal passage is also partially open in the cover plate region and thus forms a widened suction opening (25).

## Revendications

1. Masque laryngé (1) approprié pour l'introduction à travers le pharynx médian via l'épiglotte, comprenant un panneau supérieur (12), une tubulure d'introduction de tube (11), une embouchure de fenêtre respiratoire (17) et une fenêtre oesophagienne (18) ainsi qu'un manchon gonflable, entourant le panneau supérieur sur le côté ventral, sachant que le panneau supérieur s'étend de manière proximale sur tout le manchon, **caractérisé en ce que** la fenêtre oesophagienne (18) sort du panneau supérieur devant l'extrémité proximale du panneau supérieur, sur le côté dorsal, et est dirigée via le manchon (13) dans le panneau supérieur en tant que canal ouvert (20), sachant que des deux côtés de ce canal (20) et au moins à proximité de celui-ci dans le sens axial, des moyens de renforcement (21, 21', 21") sont présents, lesquels agissent contre une flexion possible de la pointe proximale du masque laryngé (4).

2. Masque laryngé selon la revendication 1, **caractérisé en ce que** le manchon (13) est réduit de la profondeur du canal (20) au niveau de la pointe proximale (4) du masque laryngé (1) par rapport au diamètre intérieur.

3. Masque laryngé selon la revendication 1, **caractérisé en ce que** le manchon (13) est interrompu au niveau de la pointe proximale (4) du masque laryngé.

4. Masque laryngé selon la revendication 2, **caractérisé en ce que** le fond (24) du canal (20) est formé par la partie ventrale de la fenêtre oesophagienne (18).

5. Masque laryngé selon la revendication 1, **caractérisé en ce que** le manchon (13) est délimité sur le côté du canal (20) par des parois de renforcement (21') au tracé ventro-dorsal et en cela, la pointe (4) du masque laryngé (1) est renforcée contre la flexion.

6. Masque laryngé selon la revendication 5, **caractérisé en ce que** les parois de renforcement (21') sont plus épaisses que les parties adjacentes du manchon (13).

7. Masque laryngé selon la revendication 5, **caractérisé en ce que** les parois de renforcement (21') sont disposées parallèlement au canal (20) en étant décalées d'une distance.

8. Masque laryngé selon la revendication 7, **caractérisé en ce qu'**un épaulement respectif en tant qu'élargissement de canal est présent entre le canal (20) et les parois de renforcement (21') des deux côtés du canal, en face du fond (24) du canal (20).

9. Masque laryngé selon la revendication 8, **caractérisé en ce qu'**au moins une nervure de renforcement (21") respective supplémentaire est formée sur les épaulements décalés.

10. Masque laryngé selon la revendication 1, **caractérisé en ce que** la profondeur du canal (20) fait au moins presque un à deux tiers du diamètre de la fenêtre oesophagienne (18) .

11. Masque laryngé selon la revendication 7, **caractérisé en ce que** des points d'affaiblissement (23) sont formés sur le passage du manchon (13) au panneau supérieur (12) sur l'extrémité distale de - l'élargissement de canal (22).

12. Masque laryngé selon la revendication 1, **caractérisé en ce que** les parois de renforcement (21') sont dirigées plus loin des deux côtés de la partie de pointe du manchon le long du côté intérieur dorsal du manchon.

13. Masque laryngé selon la revendication 1, **caractérisé en ce que** la fenêtre oesophagienne est également partiellement ouverte au niveau du panneau supérieur et forme ainsi une ouverture d'aspiration élargie (25).
